# EUROPEAN PATENT SPECIFICATION

(11) **EP 1 696 867 B1**
(45) Date of publication and mention of the grant of the patent: **15.01.2014**
(21) Application number: 04815091.6
(22) Date of filing: 17.12.2004
(51) Int. Cl.: A61K 8/22, A61K 8/81, A61Q 11/00

(54) **Oral care delivery system comprising a bleaching composition**
Mundpflegesystem enthaltend eine Bleichzubereitung
Système d'application de soin buccal contenant ume composition de blanchîment

(30) Priority: 17.12.2003 US 530397 P; 17.12.2003 US 530387 P; 17.12.2003 US 530217 P
(43) Date of publication of application: 06.09.2006
(73) Proprietor: The Procter & Gamble Company, Cincinnati, OH 45202 (US)
(72) Inventor: SCOTT, Douglas, Craig, Loveland, OH 45140 (US); GHOSH, Chanchal, Kumar, West Chester, OH 45069 (US)
(74) Representative: Joos, Uta Susanne
(86) International application number: PCT/US2004/042974
(87) International publication number: WO 2005/058268

(56) References cited:
- WO-A-98/55044
- WO-A-02/074275
- WO-A-03/024415
- US-A- 5 041 280
- US-A- 5 997 764
- US-A1- 2003 180 299
- US-B1- 6 409 993
- US-B1- 6 649 147

## Description

### TECHNICAL FIELD

The invention relates to a delivery system comprising a bleaching composition and an integral carrier, selected from a strip of material, a dental tray, and/or a sponge material.

### BACKGROUND OF THE INVENTION

Dental products by which various cosmetic and/or therapeutic actives are delivered to teeth and the oral cavity are known. Examples of such products include: brushing aids such as dentifrice products for delivery of oral care actives such as polyphosphates or fluorides; mouthwashes containing breath fresheners or antibacterial actives; and whitening strips for the delivery of bleaching actives to the teeth. In particular the use of a dental strip has been recognized as a convenient and inexpensive way to deliver cosmetic and therapeutic benefits to the teeth and mucosal surfaces of the oral cavity. For example, dental whitening strips, where a whitening composition is applied to a strip and thereafter applied to the teeth to achieve sustained contact between the teeth and the whitening composition, are known. See U.S. Pat. Nos. 6,136,297; 6,096,328; 5,894,017; 5,891,453; 5,879,691; and WO 98/55044 all to Sagel, et al., and U.S. Pat. Nos. 5,989,569 and 6,045,811 both to Dirksing, et al., and US 6649147; WO 02/074275 all assigned to The Procter & Gamble Company.

Compositions for bleaching tooth surfaces are disclosed in WO 02/024415, US 5041280, US 6409993 and US 2003/0180229.

The prior art has generally attempted to increase whitening efficacy by, among other things, increasing the level of the bleaching agent in the compositions. This approach, however, presents several problems. First the subject may experience increased irritation and/or sensitivity which may be associated with using an increased amount of a bleaching agent. Furthermore, some regulatory authorities and legislation in various geographies throughout the world do not allow bleaching agents to be used in products at levels above certain concentrations.

Therefore, despite the above known approaches for the treatment of oral conditions, especially for the whitening of teeth, a need still exists for providing products with improved bleaching efficacy. The present invention overcomes some of the limitations of the prior art.

### SUMMARY OF THE INVENTION

The present invention relates to an oral care delivery system (10) comprising:
a) an integral carrier (12) selected from a strip of material, a dental tray, sponge material and mixtures thereof and
b) a bleaching composition (14) comprising:
   i) from 0.1 % to 20% of a bleaching agent;
   ii) from 30% to 75% of a water insoluble solid phase;
   iii) from 20% to 75% of a water soluble liquid phase; and
   iv) from 0.1 % to 10% of an associative thickener;
   wherein the ratio of the water insoluble solid phase to the water soluble liquid phase is from 1:4 to 4: I and wherein the water insoluble solid phase is selected from polyethylene, polypropylene, polyisoprenes, copolymers thereof, and mixtures thereof.

The delivery system may comprise: a first layer of a strip material and a second layer comprising the above composition, whereby the bleaching agent is releaseably associated to the strip material.

### BRIEF DESCRIPTION OF THE DRAWINGS

While the specification concludes with claims that particularly point out and distinctly claim the present invention, it is believed that the present invention will be understood better from the following description of embodiments, taken in conjunction with the accompanying drawings, in which like reference numerals identify identical elements. The integral carrier is selected from a strip of material, dental tray, sponge material, and mixtures thereof. In one embodiment of the present invention, the integral carrier comprises a strip of material. The strip of material is attached to the teeth via the attachment means. The attachment means may be the present composition or an adhesive composition separate from the present composition or via an attachment means that is part of the integral carrier, e.g. the integral carrier may optionally be of sufficient size and/or width and have sufficient adhesiveness, that, once applied, the integral carrier overlaps with the oral soft tissues rendering more of the teeth surface available for bleaching.

Without intending to limit the invention, the strip of material embodiment is described in more detail below:
FIG. 1 is a perspective view of a substantially flat strip of material having rounded comers;
FIG. 2 is a perspective view of an embodiment of the present invention, disclosing the strip of FIG. 1 upon which a second layer composition comprising a composition described herein wherein the bleaching agent is releaseably associated with the integral carrier and/or the present composition;
FIG. 3 is a cross-sectional view, taken along section line 3-3 of FIG. 2, showing an example of the strip of material having a thickness less than that of the second layer coated thereon;
FIG. 4 is a cross-sectional view, showing an alternative embodiment of the present invention, showing shallow pockets in the strip of material, which act as reservoirs for additional amounts of the second layer coated on the strip;
FIG. 5 is a cross-sectional plan view, showing an alternative embodiment for applying the second layer composition to adjacent teeth having the strip of material of the present invention conforming thereto and adhesively attached to the teeth by means of the second layer composition located between the teeth and the strip of material;
FIG. 6 is a cross-sectional elevation view of a tooth, taken along section line 6-6 of FIG. 5, showing the strip of material of the present invention conforming to and adhesively attached to the teeth by means of the second layer composition located between the teeth and strip of material;
FIG. 7 is a cross-sectional plan view, similar to FIG. 5, showing a strip of material of the present invention conforming to the teeth and the adjoining soft tissue and adhesively attached to both sides of the teeth by means of the second layer composition located between the teeth and the strip of material;
FIG. 8 is a cross-sectional elevation view, taken along section line 8-8 of FIG.7, showing a strip of material of the present invention conforming to both the tooth and the adjoining soft tissue and adhesively attached to both sides of the tooth by means of the second layer composition located between the teeth and the strip of material;
FIG. 9 is a perspective view of an alternative embodiment of the present invention, disclosing the strip of material coated with a second layer composition of FIG. 2 for treating teeth and adjoining soft tissue having a release liner;

FIG. 10 is a cross-sectional view of an alternative embodiment of the present invention, taken along section line 10-10 of FIG. 9, showing a release liner attached to the strip of material by the second layer composition on the strip of the material.

### DETAILED DESCRIPTION

### Definitions

By "oral care composition" or "oral composition" as used herein is meant a product which is not intentionally swallowed for purposes of systemic administration of therapeutic agents, but is retained in the oral cavity for a sufficient time to contact the dental surfaces for purposes of whitening efficacy.

By "safe and effective amount" as used herein is meant an amount of a component, high enough to significantly (positively) modify the condition to be treated or to effect the desired whitening result, but low enough to avoid serious side effects (at a reasonable benefit/risk ratio), within the scope of sound medical/dental judgment. The safe and effective amount of a component, will vary with the particular condition (e.g., to effect whitening) being treated, the age and physical condition of the patient being treated, the severity of the condition, the duration of treatment, the nature of concurrent therapy, the specific form employed, and the particular vehicle from which the component is applied.

By "a sufficient period of time to achieve whitening" as used herein means that the composition is used or worn by the subject or the subject is instructed to use or wear the composition for greater than about 2 minutes, in another embodiment from about 2.5 minutes to about 12 hours (e.g overnight treatment), in another embodiment from about 3 minutes to about 120 minutes, in yet another embodiment from about 5 minutes to about 40 minutes, per application and may be applied from 1 to 7 times per day. Additionally, the length of treatment to achieve the desired benefit, for example, tooth whitening, may last from about one day to about six months, in another embodiment from about one day to about 28 days, and in another embodiment from about 7 to about 28 days. The optimal duration and frequency of application will depend on the desired effect, the severity of any condition being treated, the health and age of the user and like considerations.

By "cm" as used herein means centimeter. By "mm" as used herein means millimeter.

All percentages and ratios used hereinafter are by weight of total composition, unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

Without being bound by theory, the present invention may increase the "effective" concentration of the bleaching agent on the surface of the teeth due to the replacement of some of the aqueous phase with a water insoluble solid phase. In one embodiment this may make the composition more hydrophobic. Therefore, increased speed of whitening and/or increased efficacy of the bleaching agent may be achieved, even though the same or lower total level of the bleaching agent is used. The present invention, therefore, at a given total concentration of bleaching agent, may require fewer applications to get the same degree of whitening or may require a lower gel load to get the same degree of whitening.

### Bleaching Agent

The compositions comprise a safe and effective amount of a bleaching agent. Generally the level of the bleaching agent is dependent on the available oxygen or chlorine respectively that the molecule is capable of providing to bleach the stain. The bleaching agent is generally used in compositions of the present invention at levels from 0.1 % to 20%, in another embodiment from 0.5 to 9% and in another embodiment from 3% to 8%, and in yet another embodiment from 4% to 6%, by weight of the composition, of the bleaching agent. In one embodiment the bleaching agent is surprisingly more effective when used at lower levels generally from 0.5% to 3%, in another embodiment from 0.5% to 1.5% by weight of the composition.

Bleaching agents are included in the compositions of the present invention. In one embodiment bleaching agents are selected from the group consisting of peroxides, metal chlorites, perborates, percarbonates, peroxyacids, persulfates, compounds that form the preceding compounds in situ, and combinations thereof. Suitable peroxide compounds include hydrogen peroxide, urea peroxide, calcium peroxide, carbamide peroxide, and mixtures thereof. In one embodiment the bleaching agent is carbamide peroxide. Suitable metal, chlorites include calcium chlorite, barium chlorite, magnesium chlorite, lithium chlorite, sodium chlorite, potassium chlorite, and mixtures thereof. Additional bleaching agents also include hypochlorite and chlorine dioxide. In one embodiment the bleaching agent is selected from sodium chlorite, peroxide, sodium percarbonate, oxones, and mixtures thereof. The starting bleach agent can be aqueous or solid material.

### Water Insoluble Solid Phase

The bleaching composition comprises a safe and effective amount of a water insoluble solid phase. Generally the water insoluble solid phase comprises water insoluble organic (e.g. particulate) additives. The water insoluble solid phase is generally water insoluble, non-toxic to the user and chemically stable and compatible with the bleaching agent (e.g. not cause substantial decomposition of the bleach agent) and the other ingredients present in the composition. The bleaching composition comprises from 30% to 75% in another embodiment from 35% to 65% and in another embodiment from 45% to 60% by weight of the composition, of the water insoluble solid phase. is

The water insoluble solid phase is selected from the group consisting of polyethylene, polypropylene, polyisoprenes, copolymers thereof, and mixtures thereof.

Various dyes, colorants, pigments, and mixtures thereof can be optionally added to the present composition to give the compositions herein colored appearance. Any color, desired by the consumer, can be used, including dyes or pigments that provide a color similar to the color of natural teeth. One advantage of colored compositions is that it allows the user to see if the composition covers their teeth evenly and completely, since the degree of coverage on the teeth is easier to see with a colored composition.

The average particle size of the water insoluble solid phase, especially if an integral carrier (e.g. strip) is used with the composition, is generally less than 200 microns. In one embodiment, the average particle size of the water insoluble solid phase is from 5 to 100, in another embodiment from 5 to 80, in yet another embodiment from 10 to 60, and in yet another embodiment from 10 to 30 microns.

In one embodiment the water insoluble solid phase is compatible with the bleaching agent, which means that the water insoluble solid phase will not cause significant decomposition of the bleach agent. In another embodiment "compatible with the bleaching agent" means that a safe and effective amount of a stabilizer is added to the water insoluble solid phase or to the composition of this invention. In another embodiment the level of optional stabilizer is from 0.001 % to 15%, and in another embodiment from 0.01% to 10%, in another embodiment from 0.5 % to 5%, in even another embodiment from 1% to 3%, by weight of the composition.

Suitable optional stabilizers, such as chelants or non-chelant stabilizers as known in the art, may be selected from the group consisting of tartaric acid and pharmaceutically acceptable salts thereof; citric acid and salts thereof such as alkali metal citrates; pyrophosphate ion source; polyphosphates (e.g., tripolyphosphate, hexametaphosphate); diphosphonates (e.g., EHDP; AHP); EDTA; and mixtures thereof; and in another embodiment may be selected from the group consisting of sodium citrate, potassium citrate, disodium tartrate, dipotassium tartrate, pyrophosphate ion source, sodium potassium tartrate, disodium hydrogen tartrate, potassium hydrogen tartrate, disodium dihydrogen pyrophosphate, tetrasodium pyrophosphate, tetrapotassium pyrophosphate, and mixtures thereof.

### Water Soluble Liquid Phase

The bleaching composition comprises a safe and effective amount of a water soluble liquid phase. The level of the water soluble liquid phase is from 20% to 75%, in another embodiment from 35% to 70%, in another embodiment from 45 % to 65%, by weight of the composition.

The water soluble liquid phase is generally selected from the group consisting of water, polyalkylene glycols with molecular weights from 200 to 20,000, humectants, and mixtures thereof. The water soluble liquid phase can comprise water miscible components such as polyalkylene glycols, humectants, and mixtures thereof. Humectants generally include edible polyhydric alcohols such as glycerin, sorbitol, xylitol, butylene glycol, polyethylene glycol, and propylene glycol, and mixtures thereof. In one embodiment the water soluble liquid phase is water. In one embodiment the composition comprises at least 10% water, in another embodiment at least 20% by weight of the composition, of water.

### Ratio of Water Insoluble Solid Phase to Water Soluble Liquid Phase

The ratio of the water insoluble solid phase to the water soluble liquid phase is from 1:4 to 4:1, in another embodiment is from 1:2.5 to 2.5:1, in another embodiment from 1:1.5 to 1.5:1, and in yet another embodiment is from 1:1.25 to 1.25:1.

### Whitening Index

In one embodiment, the bleaching composition has a Whitening Index of from 0.5 to 4, in another embodiment from 1 to 4, in yet another embodiment from 1.3 to 3.5, and in another embodiment from 1.5 to 3. The Whitening Index is calculated as follows:
Db (for composition A) minus Db (for composition B) at treatment day 1, 2, 3 or 4 wherein composition A and composition B have the same concentration of bleaching agent, by weight of the composition, and composition A is a composition of the present invention, having the requisite amount of water insoluble solid phase and water soluble liquid phase at a ratio of from about 1:4 to about 4:1, etc., and composition B does not.

Db for composition A is calculated as follows: 6-8 extracted human molars are cleaned and are mounted into Lego® blocks, and the front side of each molar is labeled to identify each molar. The molars are re-hydrated overnight in either water or phosphate buffer solution. Thereafter, the molars are removed from solution and then 0.03 to 0.1g of composition A is applied to the front surface of each molar. The molars are then placed in a 37 degree C incubator during the duration of the treatment. Each molar is treated with composition A for 30 minutes twice daily over a 4-day study period. After 30 minutes treatment time, the molars are removed from the incubator and are rinsed with distilled water to remove any residual composition. The molars are placed in the water or buffer solution in between each treatment. Two to four hours are allowed between each treatment period.

Digital images of the molars are obtained pretreatment (baseline) and after each total daily treatment (total daily treatment is 1 hour). Digital images of the molars are captured on computer using a high resolution digital camera (HC 1000 CCD®) manufactured by Fuji, Japan. Theses images are analyzed to derive numerical values for average tooth color in terms of standard CIELAB¹ three dimensional color space describing lightness/brightness from blue to yellow (b). Treatment efficacy after each day of treatment, b (calculated as the average b for each molar tested) is compared to the baseline b (calculated as the average b for each molar tested) for color measures b denoted as Db. In one embodiment the baseline b value of the extracted human molars ranges from about 10 to about 20, in another embodiment from about 12 to about 16.
¹ Commission Internationale de l'Eclairage L*a*b* capable of representing all possible colors using three variables, a luminence -L*; and color values on a red-green axis- a*; and a blue-yellow axis -b*.

The Whitening Index can be calculated at day 1 or 2 or 3 or 4, or it can an average of the Db from days 1 to 4.

Db for composition B is calculated the same way as for composition A except that composition B is used instead of composition A.

### Thickening Agents

The compositions comprise an associative thickener. The thickening agent (or viscosity modifier) functions to increase retention of the composition on the teeth. The viscosity modifier may further function to inhibit settling and separation of components or control settling in a manner that facilitates redispersion and may control flow properties of the composition. A viscosity modifier is particularly useful to keep bleach agents or other oral care active agents, that are in particulate form, suspended within the bleaching compositions. The thickening agent herein can also serve as the adhesive means discussed herein below.

The thickening agent (viscosity modifier) is present at a level of from 0.1 % to 10%, and in another embodiment from 0.4% to 5%, and in yet another embodiment from 1% to 3%, by weight of the composition.

The thickening agent is an associative thickener , such as a hydrophobically modified alkali soluble acrylic emulsion or a hydrophobically modified nonionic polyol polymer, i.e., a hydrophobically modified urethane polymer, hydrophobically modified ethoxylated urethane polymer or combinations thereof. Associative thickeners may increase the retention or adhesion of compositions herein and/or integral carriers on the tooth surfaces, may slow the erosion of the compositions once applied on the tooth surfaces, and may improve the release of the compositions from the optional release liner disclosed herein.

Hydrophobically modified polyacrylic acid polymers are described, for example, in U.S. Pat. Nos. 3,915,921, 4,421,902, 4,509,949, 4,923,940, 4,996,274, 5,004,598, and 5,349,030. These polymers have a large water-loving hydrophilic portion (the polyacrylic acid portion) and a smaller oil-loving hydrophobic portion (which can be derived from a long carbon chain acrylate ester). Representative higher alkyl acrylic esters are decycl acrylate, lauryl acrylate, stearyl acrylate, behenyl acrylate and melissyl acrylate, and the corresponding methacrylates. It should be understood that more than one carboxylic monomer and more than one acrylate ester or vinyl ester or ether or styrenic can be used in the monomer charge. The polymers can be dispersed in water and neutralized with base to thicken the aqueous composition, form a gel, or emulsify or suspend a deliverable. Useful polymers are sold as Carbopol® 1342 and 1382, and Carbopol® ETD 2020, and Pemulen® TR-1, TR-2, 1621, and 1622, all available from BF Goodrich. The carboxyl containing polymers are prepared from monomers containing at least one activated vinyl group and a carboxyl group, and would include copolymers of polymerizable carboxylic monomers with acrylate esters, acrylamides, alkylated acrylamides, olefins, vinyl esters, vinyl ethers, or styrenics. The carboxyl containing polymers have molecular weights greater than about 500 to as high as several billion, or more, usually greater than about 10,000 to 900,000 or more.

Also useful are interpolymers of hydrophobically modified monomers and steric stabilizing polymeric surface active agents having at least one hydrophilic moiety and at least one hydrophobic moiety or a linear block or random comb configuration or mixtures thereof. Examples of steric stabilizers which can be used are Hypermerl, which is a poly(12-hydroxystearic acid) polymer, available from Imperial Chemical Industries Inc. and Pecosil®, which is a methyl:-3-polyethoxypropyl siloxaneΩ.-phosphate polymer, available from Phoenix Chemical, Somerville, N.J. These are taught by U.S. Pat. Nos. 4,203,877 and 5,349,030.

Other associative thickeners include Rohm and Haas (such as Acrysol® ICS-1 and Aculyn® 22 and 28 thickeners, which are hydrophobically modified alkali-soluble acrylic polymer emulsions and Aculyn® 44 and 46 thickener, which is a hydrophobically modified nonionic polyol). In one embodiment the associative thickener is Carbopol® and/or Pemulen® polymers. The choice of the specific polymer to be employed will depend upon the desired rheology of the composition, and the identity of other compositional ingredients.

Other associative thickeners are discussed in greater detail in US 5,997,764, BF Goodrich. In one embodiment mixtures of hydrophobically modified carbomers with carbomers can be used.

Combination of Integral Carrier and Composition The present invention relates to a delivery system comprising an integral carrier and a bleaching composition. In one embodiment the delivery system comprises: a first layer of a strip of material; a second layer comprising the above compositions, whereby the bleaching agent is releasably associated with the composition and/or the strip of material. The present invention delivers whitening benefits to the oral cavity by directly applying the integral carrier to the teeth.

### I. First Layer

The first layer of the present invention comprises an integral carrier including a strip of material, dental tray, a sponge material, and mixtures thereof. In one embodiment the integral carrier is a strip of material.

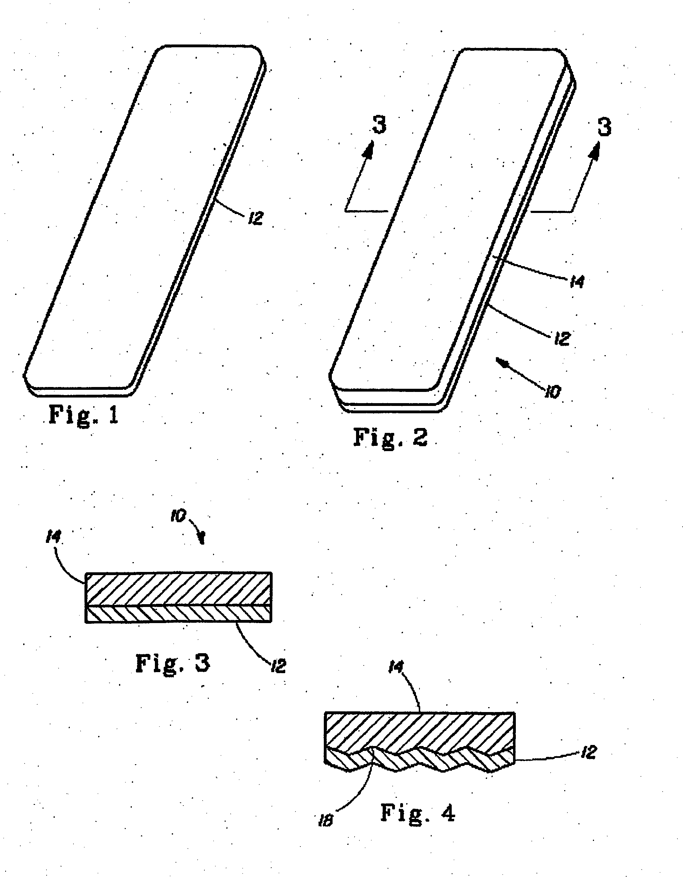

Referring now to the drawings, and more particularly to FIGS. 1 and 2, there is shown a first embodiment of the present invention, generally indicated as 10, representing a delivery system for delivering bleach actives to the teeth and the oral cavity. Delivery system 10 has a strip of material 12, which is substantially flat, preferably with rounded corners.
Releasably applied onto said strip of material 12 is a second layer composition 14. Second layer composition 14 is, in one embodiment, a homogenous, and may be uniformly and continuously coated onto strip of material 12, as shown in FIG. 3. However, second layer composition 14 may alternatively be a continuous coating of second layer composition 14 along a longitudinal axis of a portion of strip of material 12. In addition second layer composition may be laminated or layered wherein the bleaching agent and/or adhesive means may not be present in all layers or laminates. In addition second layer composition may be an amorphous mixture of compositions wherein the bleaching agent and/or adhesive means may not be present in all phases of the amorphous mixture. In addition second layer composition may be applied as stripes, spots, and/or other patterns of the same or different compositions, wherein the bleaching agent and/or adhesive means may not be present in all stripes, spots, and/or other patterns of compositions.

As shown in FIG. 4 in an alternative embodiment, strip of material 12 may have shallow pockets 18 formed therein. When second layer composition 14 is coated on a strip of material 12, additional second layer composition 14, if present, fills shallow pockets 18 to provide reservoirs of second layer composition 14.

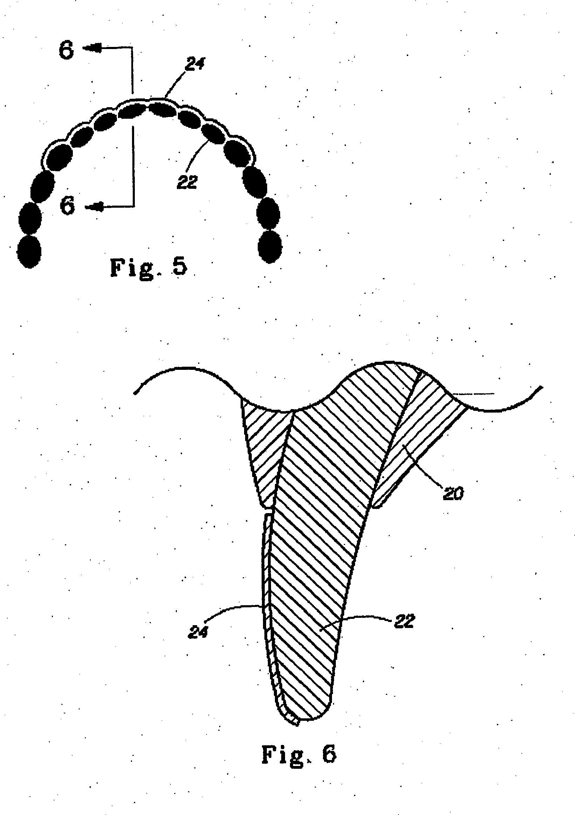

FIGS. 5 and 6 show a delivery system 24 of the present invention applied to the surface of a tooth and plurality of adjacent teeth. Embedded in adjacent soft tissue 20 are a plurality of adjacent teeth 22. Adjacent soft tissue herein defined as soft tissue surfaces surrounding the tooth structure including: papilla, marginal gingival, gingival sulculus, inter dental gingival, and gingival gum structure on lingual and buccal surfaces up to and including muco-gingival junction on the pallet.

In both FIGS. 5 and 6, delivery system 24 represents strip of material 12 and second layer composition 14, with second layer composition 14 on the side of strip material 12 facing tooth 22. Second layer composition 14 may be pre-applied to strip of material 12, or may be applied to strip of material 12 by the user prior to application to the teeth. In an alternate embodiment, the second layer composition may be applied directly to teeth 22 by the user and then covered by a strip of material 12. In any case, strip of material 12 has a thickness and flexural stiffness such that it can conform to the contoured surfaces of tooth 22 and to adjacent soft tissue 20. In one embodiment, the strip of material has sufficient flexibility to form to the contours of the oral surface, the surface being a plurality of adjacent teeth. The strip of material is also readily conformable to tooth surfaces and to the interstitial tooth spaces without permanent deformation when the delivery system is applied. The delivery system can be applied without significant pressure.

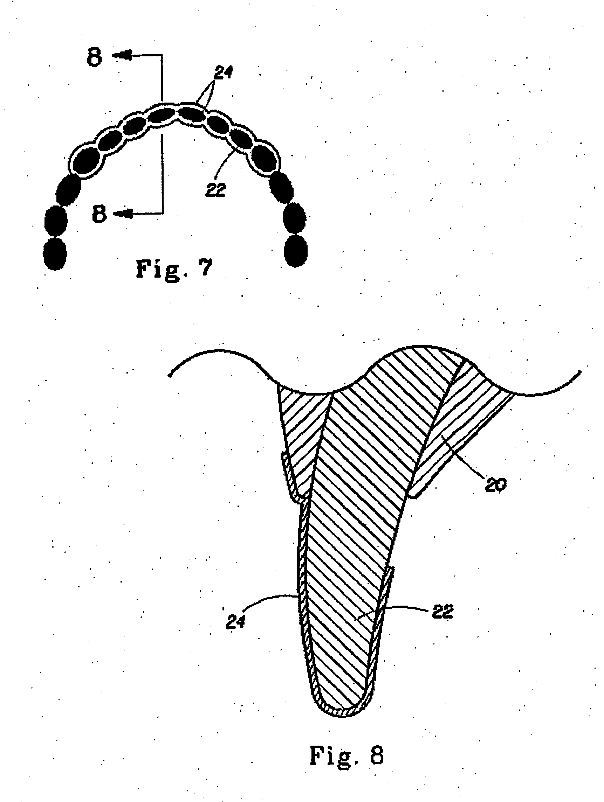

FIGS. 7 and 8 show a delivery system 24 of the present invention applied to both front and rear surfaces of a plurality of adjacent teeth 22 as well as to adjacent soft tissue 20. Delivery system 24 represents strip of material 12 and second layer composition 14, with second layer composition 14 on the side of strip of material 12 facing tooth 22.

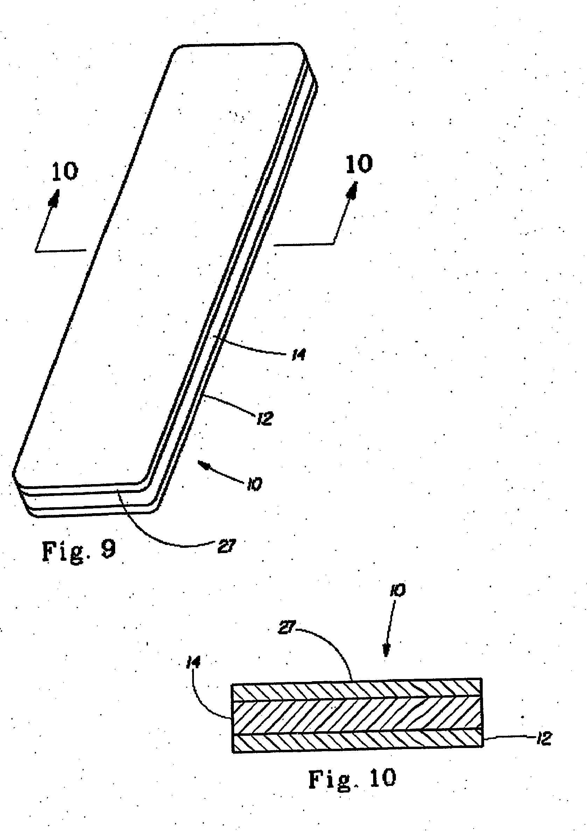

FIGS. 9 and 10 shows an optional release liner 27. Release liner 27 is attached to strip of material 12 by second layer composition 14. Second layer composition 14 is on the side of strip material 12 facing release liner 27. This side is applied to the tooth and gum surfaces once release liner 27 is removed.
In one embodiment the first layer of the delivery system of the present invention is comprised of a strip of material. Such first layer materials are described in more detail in U.S. Pat. Nos; 6,136,297; 6,096,328; 5,894,017; 5,891,453; and 5,879,691, all to Sagel, et al., and all assigned to The Procter & Gamble Company, and in U.S. Pat. Nos. 5,989,569 and 6,045,811 both to Dirksing, et al., and both assigned to The Procter & Gamble Company.

The strip serves as a protective barrier for the bleach. It prevents leaching and/or erosion of the second layer by for example, the wearer's tongue, lips, and saliva. This allows the active in the second layer to act upon the hard surfaces of the oral cavity for an extended period of time, from several minutes to several hours.

The strip material may comprise polymers, natural and synthetic woven materials, non-woven material, foil, paper, rubber and combinations thereof. The strip material may be a single layer of material or a laminate of more than one layer. Regardless of the number of layers, the strip of material is, in one embodiment, substantially water insoluble. The strip may also be water impermeable. In one embodiment the material is any type of polymer or combination of polymers that meet the required flexural rigidity and are compatible with oral care substances. Suitable polymers include, but are not limited to, polyethylene, ethylvinylacetate, polyesters, ethylvinyl alcohol and combinations thereof. Examples of polyesters include Mylar® and fluoroplastics such as Teflon®, both manufactured by Dupont. In one embodiment the material is polyethylene. The strip of material is generally less than about 1 mm (millimeter) thick, in one embodiment less than about 0.05 mm thick, in yet another embodiment from about 0.001 to about 0.03 mm thick. A polyethylene strip of material is generally less than about 0.1 mm thick and in one embodiment from about 0.005 to about 0.02 mm thick.

The shape of the strip of material is any shape and size that covers the desired oral surface. In one embodiment the strip has rounded corners to avoid irritation of the soft tissue of the oral cavity. "Rounded corners," means not having any sharp angles or points. In one embodiment, the length of the strip material is from about 2 cm (centimeter) to about 12 cm, in another embodiment from about 4 cm to about 9 cm. The width of the strip material will also depend on the oral surface area to be covered. The width of the strip is generally from about 0.5 cm to about 4 cm, in one embodiment from about 1 cm to about 2 cm. In yet another embodiment, the strip may be worn as a patch on one or several teeth to treat a localized condition.

The strip material may contain shallow pockets. When the composition is coated on a strip of material, bleach agent and/or oral care actives fill shallow pockets to provide reservoirs of additional bleach agent and/or oral care actives. Additionally the shallow pockets help to provide texture to the delivery system. In one embodiment the strip material will have an array of shallow pockets. Generally the shallow pockets are approximately 0.4 mm across and about 0.1 mm deep. When shallow pockets are included in the strip of material and the compositions herein are applied to it in various thicknesses, the overall thickness of the delivery system is less than about 1 mm. In one embodiment the overall thickness is less than about 0.5 mm.

Flexural stiffness is a material property that is a function of a combination of strip thickness, width and material modulus of elasticity. This test is a method for measuring the rigidity of polyolefin film and sheeting. It determines the resistance to flexure of a sample by using a strain gauge affixed to the end of a horizontal beam. The opposite end of the beam presses across a strip of the sample to force a portion of the strip into a vertical groove in a horizontal platform upon which the sample rests. A microammeter wired to the strain gauge is calibrated in terms of deflection force. The rigidity of the sample is read directly from the microammeter and expressed as grams per centimeter of the sample strip width. In the present invention, the strip of material has a flexural stiffness of less than about 5 grams/cm as measured on a Handle-O-Meter, model #211-300, available from Thwing-Albert Instrument Company of Philadelphia, PA as per test method ASTM D2923-95. In one embodiment the strip has a flexural stiffness less than about 3 grams/cm, in another embodiment less than about 2 grams/cm and in yet another embodiment from about 0.1 to about 1 grams/cm. Generally, the flexural stiffness of the strip of material is substantially constant and does not change during normal use. For example, the strip of material does not need to be hydrated for the strip to achieve the low flexural stiffness in the above-specified ranges.

This relatively low stiffness enables the strip of material to cover the contours of the oral surface with very little force being exerted. That is, conformity to the contours of the oral surface of the wearer's mouth is maintained because there is little residual force within the strip of material to cause it to return to its shape just prior to its application to the oral surface, i.e, substantially flat. The strip of material's flexibility enables it to contact soft tissue over an extended period of time without irritation. The strip of material does not require continuous pressure for retention against the oral surface.

In one embodiment the delivery systems herein comprise an adhesion means and are capable of adhesion to oral surfaces especially the teeth. This adhesion means may be provided by the present compositions herein or the adhesion means is provided independently of the compositions herein (for example the adhesion means is a separate phase from the compositons herein where the compositions may or may not also have an adhesive means). In once embodiment the strip of material is held in place on the oral surface by adhesive attachment provided by the present compositions. The viscosity and general tackiness of the present compositions to dry surfaces cause the strip to be adhesively attached to the oral surface without substantial slippage from the frictional forces created by the lips, teeth, tongue, and other oral surfaces rubbing against the strip of material while talking drinking, etc. However, this adhesion to the oral surface is low enough to allow the strip of material to be easily removed by the wearer by simply peeling off the strip of material using one's finger. The delivery system is easily removable from the oral surfaces without the use of an instrument, a chemical solvent or agent or excess friction.

In another embodiment the strip of material is held in place on the oral surface by adhesive means and attachment provided by the integral carrier itself. In one embodiment the strip of material can extend, attach, and adhere to the oral soft tissue. Alternatively, an adhesive can be applied to that portion of the strip of material that will attach the delivery systems to the oral soft tissue. In another embodiment the strip of material is held in place by an adhesion means that is independent of the composition of the present inventions herein, as disclosed in WO 03/015656, published Feb. 27, 2003, SmithKline Beecham.

Examples of adhesion means being provided independent of the compositions herein include the following.

In one embodiment, the composition and an adhesive material may be deposited in separate discrete locations in relation to the strip surface. In one embodiment the composition and adhesive may be deposited on the surface of the strip in respective spatially separated places on the surface. For example the adhesive may be deposited in places on the strip surface that enable part of the strip to stick to an oral surface adjacent to a tooth surface, e.g. a gum surface, so that another part of the strip on which the composition is deposited or into which it is impregnated may contact the tooth surface.

Alternatively the adhesive and composition may be spatially separated but both in locations that enable the adhesive and composition to contact the same type of tissue, e.g. tooth or gum surface, respective discrete spots or patches on the surface, that are relatively small. For example parallel lines of the adhesive and composition, one or more patches of composition bordered partly or completely surrounded by a border of the adhesive, a single large patch covering substantially the entire surface of the strip and bordered partly or completely by a line of the adhesive. Adhesive may be deposited on one or more patch bordered partly or completely surrounded by a border of the composition.

In another embodiment the composition and/or adhesive may be encapsulated. Encapsulation may for example be in micro-capsules, or macro-capsules. Methods of micro-encapsulation are known, for example in which a droplet of a substance in a liquid phase is enclosed within a layer of an encapsulation material, and then separated from the liquid. Such capsules may be deposited on or adjacent the surface of the integral carrier, and may for example be burst physically or chemically, e.g. by pressure e.g. as the strip is applied to the tooth surface or by subsequent bit action, by breaching of the capsule wall under the action of the temperature, moisture, pH, chemicals or enzymes in the mouth environment etc. For example respective capsules of composition and adhesive may be attached to the surface of the strip, e.g. by means of a second adhesive or by embedding the capsules in the strip of material. For example a thin layer of the adhesive may be deposited on the surface of the strip, and capsules of the composition may be embedded at least partly if not completely within this adhesive layer, or may sit upon the surface of this adhesive layer.

In another embodiment the adhesive may be provided in granules, e.g. pellets or micropellets, which may release their content under the influence of the mouth environment, for example moisture, chemicals or enzymes in the mouth, and may be coated to achieve this release. Methods of granulation and palletizing are known, as are coating polymers such as the know Eudragit™ polymers which dissolve at specified pH. Such adhesive granules may be deposited on or adjacent the surface of the strip. For example capsules and/or granules of adhesive may be located substantially uniformly over the strip surface, or alternatively respective capsules and/or granules of adhesive may be situated at separate respective locations on the surface of the strip.

In another embodiment a layer of the composition may be deposited relatively proximal to, e.g. adjacent to and in contact with the surface, and a layer of the adhesive may be deposited relatively distal from the surface e.g. adjacent to and in contact with the underlying layer of composition. In such a construction the adhesive may stick the strip to the tooth surface, and the composition may pass through the adhesive layer, for example as the adhesive layer becomes permeable under the influence of the mouth environment. The adhesive layer may in such a construction have one or more holes passing through the layer to facilitate the passage of the composition through the adhesive layer. Alternatively for example a layer of the adhesive may be deposited relatively proximal to, e.g. adjacent to and in contact with the surface, and a layer of the composition may be deposited relatively distal from the surface e.g. adjacent to and in contact with an underlying layer of adhesive. In such a construction the layer of composition may need one or more holes passing through the layer to facilitate the passage of the adhesive through the composition. In the above constructions the passage of material from the underlying layer may be facilitated by pressure as the strip is applied to the tooth surface.

Mechanical adhesive means may also be used to provide an adhesive function, used either alone or in combination with any other adhesive device disclosed herein. In another embodiment mechanical adhesion between the strip and tooth or other oral surface is provided by the strip comprising a plastically deformable material, which can be plastically deformed by the user to conform the strip to the contours of the tooth or other oral surface, and so adhere thereto by mechanical gripping. Such gripping may be enhanced by e.g. surface effects between the strip and the surface such as formation of a partial vacuum or surface tension effects. For example the strip may have anchors on its surface, positioned at approximately the spacings of gaps between teeth, and these anchors may fit into the gaps between the teeth. For example the surface of the strip which is to contact the tooth surface may be provided with micro-suckers, that is a plurality of small cavities in the surface of the strip which can be pressed onto the tooth surface to drive air out therefrom, and thereby create a partial vacuum, so that the strip is thereafter held on the tooth surface by air pressure. Such anchors or micro-suckers may be located on the surface of a strip which is to contact the tooth surface. Such a strip may for example be stretchable, so that it can be adjusted to the spacings of gaps between an individual user's teeth. Another form of "mechanical" adhesion may be provided by a strip which shrinks in contact with the tooth surface, so it can physically grip the surface of the tooth.

When the adhesive means is provided by an adhesive, the adhesive may be any adhesive which may be used to stick materials to the tooth surface or to a surface of the oral cavity surfaces. Suitable adhesives include skin, gum and muco adhesives, and should be able to withstand the moisture, chemicals and enzymes of the oral environment for long enough for the oral care actives and/or bleach to take effect, but may be soluble and/or biodegradable thereafter. Suitable adhesives may for example comprise water soluble polymers, hydrophobic and/or non-water soluble polymers, pressure and moisture sensitive adhesives, e.g. dry adhesives which become tacky upon contact with the mouth environment, e.g. under the influence of moisture, chemicals or enzymes etc. in the mouth. Suitable adhesives include natural gums, synthetic resins, natural or synthetic rubbers, those gums and polymers listed above under "Thickening Agents", and various other tacky substances of the kind used in known adhesive tapes, those known from US-A-2,835,628.

### Second Layer

In one embodiment the second layer comprises a safe and effective amount of the bleaching composition described herein.

### Optional Release Liner

The release liner may be formed from any material which exhibits less affinity for the second layer composition than the second layer composition exhibits for itself and for the first layer strip of material. The release liner may comprise a rigid sheet of material such as polyethylene, paper, polyester, or other material, which is then coated with a nonstick type material. The release liner may be cut to substantially the same size and shape as the strip of material or the release liner may be cut larger than the strip of material to provide a readily accessible means for separating the material from the strip. The release liner may be formed from a brittle material that cracks when the strip is flexed or from multiple pieces of material or a scored piece of material. Alternatively, the release liner may be in two overlapping pieces such as a typical adhesive bandage design. A description of materials suitable as release agents is found in Kirk-Othmer, Encyclopedia of Chemical Technology, Fourth Edition, Volume 21, pp. 207-218,

### Combination of Soft/Rigid Dental

### Trays or Sponge Material (Foams) and Composition

The delivery systems may be used in combination with a dental tray. Dental trays are well known in the whitening art. The general process for preparing dental trays is known in the art. For example, an alginate impression which registers all teeth surfaces plus gingival margin is made and a stone cast is promptly made of the impression. If reservoirs are desired they are prepared by building a layer of rigid material on the stone cast on specific teeth surfaces to be treated. A dental tray is then vacuum formed from the modified cast using conventional techniques. Once formed, the tray is preferably trimmed barely shy of the gingival margin on both buccal and lingual surfaces. Enough tray material should be left to assure that all of the tooth will be covered to within about ¼ to about 1/3 mm of the gingival border upon finishing and beveling the tray periphery. In one embodiment one can scallop up and around interdental papilla so that the finished tray does not cover them. All tray edges are preferably smoothed so that the lip and tongue will not feel an edge prominence. The resulting tray, in one embodiment, provides a perfect fit of the patient's teeth optionally with reservoirs or spaces located where the rigid material was placed on the stone cast. Dental trays may comprise of soft transparent vinyl material having a preformed thickness from about 0.04 inch to about 0.06 inch. Soft material is more comfortable for the patient to wear. Harder material (or thicker plastic) may also be used to construct the tray.

Dentists have traditionally utilized three types of dental appliances for bleaching teeth. The first type is a rigid appliance which is fitted precisely to the patient's dental arches. A second type of rigid custom dental appliance is an "oversized" rigid custom dental appliance. The fabrication of rigid, custom dental appliances entails fabricating stone models of the patient's dental arch impressions, and heating and vacuum-forming a thermoplastic sheet to correspond to the stone models of a patient's dental arches. Thermoplastic films are sold in rigid or semi rigid sheets, and are available in various sizes and thickness. The dental laboratory fabrication technique for the oversized rigid dental appliance involves augmenting the facial surfaces of the teeth on the stone models with materials such as die spacer or light cured acrylics. Next, thermoplastic sheeting is heated and subsequently vacuum formed around the augmented stone models of the dental arch. The net effect of this method results in an "oversized" rigid custom dental appliance.

A third type of rigid custom dental appliance, used with less frequency, is a rigid bilaminated custom dental appliance fabricated from laminations of materials, ranging from soft porous foams to rigid, non-porous films. The non-porous, rigid thermoplastic shells of these bilaminated dental appliances encase and support an internal layer of soft porous foam.

A fourth type of dental tray replaces rigid custom dental appliances with disposable U-shaped soft foam trays, which may be individually packaged, and which may be saturated with a pre-measured quantity of the composition of the present invention. The soft foam material is generally an open celled plastic material. Such a device is commercially available from Cadco Dental Products in Oxnard, Calif. under the tradename VitalWhite™. In one embodiment these soft foam trays comprise a backing material (e.g. a closed cell plastic backing material) to minimize the elution of the bleaching agent from the device, into the oral cavity to minimize ingestion by the patient and/or irritation of the oral cavity tissues. In another embodiment the soft foam tray is encased by a nonporous flexible polymer. In another embodiment the open cell foam is attached to the frontal inner wall of the dental appliance and/or the open cell foam is attached to the rear inner wall of the dental appliance.

Those of ordinary skill in the art will readily recognize and appreciate, that the present compositions must be thick enough not to simply run out between the open cell structure of the foam and must be thin enough to slowly pass through the open cell foam over time. In other words, the open cell foam material has an internal structural spacing sized relative to the viscosity of the compositions to absorb and allow the composition to pass therethrough.

An example of a closed cell material is a closed-celled polyolefin foam sold by the Voltek division of Sekisui America Corporation of Lawrence, Mass. under the tradename Volora which is from 1/32" to 1/8" in thickness. A closed cell material may also comprise of a flexible polymeric material.

An example of an opened cell material is an open celled polyethylene foam sold by the Sentinel Foam Products division of Packaging Industries Group, Inc. of Hyannis, Mass. under the tradename Opcell which is from 1/16" to 3/8" in thickness. Other open cell foam useful herein include hydrophilic open foam materials such as hydrogel polymers (e.g Medicell™ foam available from Hydromer, Inc. Branchburg, J.J.). Open cell foam may also be hydrophilic open foam material imbibed with agents to impart high absorption of fluids, such as polyurethane or polyvinylpyrrolidone chemically imbibed with various agents.

Appliances of the above type are further described in US Patent Nos. 5,980,249, M.G. Fontenot, and US 5,575,654, M.G. Fontenot.

The above dental appliances may be designed to be disposable or reuseable. Further dental trays are disclosed in U.S. Patent 6,368,576, Steven D. Jensen, issued April 9, 2002; U.S. Patent 6,309,625 Jensen, et al., issued Oct 30, 2001; U.S. Patent 6,183,251, Dan E. Fischer, issued February 6, 2001; U.S. Patent 6,036,943, Dan E. Fischer, issued March 14, 2000; U.S. Patent 5,985,249, Dan E. Fischer, issued November 16, 1999; U.S. Patent 5,846,058, Dan E. Fischer, issued Dec 8, 1998; U.S. Patent 6,382,979, Sherrill F. Lindquist, issued May 7, 2002; U.S. Patent 5,098,303, Fischer, issued March 24, 1992, and U.S. Patent 5,855,870, Dan E. Fischer, issued January 5, 1999.

### Optional Oral Care Active Agents

The bleaching composition may optionally comprise a safe and effective amount of an oral care active agent selected from the group consisting of anticalculus agent, fluoride ion source, antimicrobial agents, dentinal desensitizing agents, anesthetic agents, antifungal agents, anti-inflammatory agents, selective H-2 antagonists, anticaries agents, nutrients, and mixtures thereof. The oral care active agent preferably contains an active at a level where upon directed use, the benefit sought by the wearer is promoted without detriment to the oral surface to which it is applied. Examples of the oral conditions these actives address include, but, are not limited to, appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, and the elimination of mouth malodor resulting from the conditions above and other causes such as microbial proliferation.

Suitable oral care actives include any material that is generally considered safe for use in the oral cavity and that provides changes to the overall appearance and/or health of the oral cavity. The level of oral care substance in the bleaching compositions is generally, unless specifically noted, from 0.01% to 50%, preferably from 0.1% to 20%, more preferably from 0.5% to 10%, and even more preferably from 1% to 7%, by weight of the composition.

Bleaching compositions may include many of the actives previously disclosed in the art. The following is a non-limiting list of oral care actives that may be used in the present invention.

### Anticaries Agents and Fluoride Ion Source

The composition may comprise a safe and effective amount of an anticaries agent, and mixtures thereof. In one embodiment the anticaries agent is selected from the group consisting of xylitol, fluoride ion source, and mixtures thereof. The fluoride ion source provides free fluoride ions during the use of the composition. In one embodiment the oral care active agent is a fluoride ion source selected from the group consisting of sodium fluoride, stannous fluoride, indium fluoride, organic fluorides such as amine fluorides, and sodium monofluorophosphate. Sodium fluoride is the fluoride ion in another embodiment. Norris et al., U.S. Patent 2,946,725, issued July 26, 1960, and Widder et al., U.S. Patent 3,678,154 issued July 18, 1972, disclose such fluoride salts as well as others that can be used as the fluoride ion source.

Preferably the instant compositions provide from 50 ppm to 10,000 ppm, more preferably from 100 to 3000 ppm, of fluoride ions in the compositions that contact dental surfaces when used with the delivery system of the present invention Anticalculus Agents

The compositions may comprise a safe and effective amount of at least one anticalculus agent. This amount is generally from 0.01% to 40% by weight of the composition, in another embodiment is from 0.1% to 25%, and in yet another embodiment is from 4.5% to 20%, and in yet another embodiment is from 5% to 15%, by weight of the composition. The anticalculus agent should also be essentially compatible with the other components of the composition.

The anticalculus agent is selected from the group consisting of polyphosphates and salts thereof; polyamino propane sulfonic acid (AMPS) and salts thereof; polyolefin sulfonates and salts thereof; polyvinyl phosphates and salts thereof; polyolefin phosphates and salts thereof; diphosphonates and salts thereof; phosphonoalkane carboxylic acid and salts thereof; polyphosphonates and salts thereof; polyvinyl phosphonates and salts thereof; polyolefin phosphonates and salts thereof; polypeptides; and mixtures thereof. In one embodiment, the salts are alkali metal salts. In another embodiment the anticalculus agent is selected from the group consisting of polyphosphates and salts thereof; diphosphonates and salts thereof; and mixtures thereof. In another embodiment the anticalculus agent is selected from the group consisting of pyrophosphate, polyphosphate, and mixtures thereof.

### Polyphosphate

In one embodiment of the present invention, the anticalculus agent is a polyphosphate. A polyphosphate is generally understood to consist of two or more phosphate molecules arranged primarily in a linear configuration, although some cyclic derivatives may be present. Linear polyphosphates correspond to (X PO₃)ₙ where n is 2 to 125, wherein preferably n is greater than 4, and X is for example sodium, potassium, etc. For (X PO₃)ₙ when n is at least 3 the polyphosphates are glassy in character. Counterions for these phosphates may be the alkali metal, alkaline earth metal, ammonium, C₂-C₆ alkanolammonium and salt mixtures. Polyphosphates are generally employed as their wholly or partially neutralized water soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials. In one embodiment the polyphosphates are those manufactured by FMC Corporation which are commercially known as Sodaphos (n ≈6), Hexaphos (n≈13), and Glass H (n≈21), and mixtures thereof. The compositions will typically comprise from 0.5% to 20%, in one embodiment from 4% to 15%, in yet another embodiment from 6% to 12%, by weight of the composition of polyphosphate.

The phosphate sources are described in more detail in Kirk & Othmer, Encyclopedia of Chemical Technology, Fourth Edition, Volume 18, Wiley-Interscience Publishers (1996), pages 685-707.

In one embodiment the polyphosphates are the linear "glassy" polyposphates having the formula:

XO(XPO₃)ₙX

wherein X is sodium or potassium; and n averages from about 6 to about 125.

In one embodiment, when n is at least 2 in either of the above polyphosphate formulas, the level of anticalculus agent is from 4.5% to 40%, in another embodiment is from 5% to 25%, and in even another embodiment is from 8% to 15%, by weight of the composition. Polyphosphates are disclosed in US 4,913,895.

### Pyrophosphate

The pyrophosphate salts useful in the present compositions include, alkali metal pyrophosphates, di-, tri-, and mono-potassium or sodium pyrophosphates, dialkali metal pyrophosphate salts, tetraalkali metal pyrophosphate salts, and mixtures thereof. In one embodiment the pyrophosphate salt is selected from the group consisting of trisodium pyrophosphate, disodium dihydrogen pyrophosphate (Na₂H₂P₂O₇), dipotassium pyrophosphate, tetrasodium pyrophosphate (Na₄P₂O₇), tetrapotassium pyrophosphate (K₄P₂O₇), and mixtures thereof. The pyrophosphate salts described in U.S. Patent 4,515,772, issued May 7, 1985, and US Pat. No. 4,885,155, issued December 5, 1989, both to Parran et al. The pyrophosphate salts are described in more detail in Kirk & Othmer, Encyclopedia of Chemical Technology, Third Edition, Volume 17, Wiley-Interscience Publishers (1982), pages 685-707

In one embodiment, the bleaching compositions comprise tetrasodium pyrophosphate. Tetrasodium pyrophosphate may be the anhydrous salt form or the decahydrate form, or any other species stable in solid form in the present compositions. The salt is in its solid particle form, which may be its crystalline and/or amorphous state, with the particle size of the salt preferably being small enough to be aesthetically acceptable and readily soluble during use.

The level of pyrophosphate salt in the compositions of the present invention is any safe and effective amount, and is generally from 1.5% to 15%, in another embodiment from 2% to 10%, and yet in another embodiment from 3% to 8%, by weight of the composition.

### Other Anticalculus Agents

Polyolefin sulfonates include those wherein the olefin group contains 2 or more carbon atoms, and salts thereof. Polyolefin phosphonates include those wherein the olefin group contains 2 or more carbon atoms. Polyvinylphosphonates include polyvinylphosphonic acid. Diphosphonates and salts thereof include azocycloalkane-2,2-diphosphonic acids and salts thereof, ions of azocycloalkane-2,2-diphosphonic acids and salts thereof (such as those which the alkane moiety has five, six or seven carbon atoms, in which the nitrogen atom is unsubstituted or carries a lower alkyl substitutent, e.g. methyl), azacyclohexane-2,2-diphosphonic acid, azacyclopentane-2,2-diphosphonic acid, N-methyl-azacyclopentane-2,3-diphosphonic acid, EHDP (ethanehydroxy-1,1,-diphosphonic acid), AHP (azacycloheptane-2,2-diphosphonic acid, a.k.a. 1-azocycloheptylidene-2,2-diphosphonic acid), ethane-1-amino-1,1-diphosphonate, dichloromethane-diphosphonate, etc. Phosphonoalkane carboxylic acid or their alkali metal salts include PPTA (phosphonopropane tricarboxylic acid), PBTA (phosphonobutane-1,2,4-tricarboxylic acid), each as acid or alkali metal salts. Polyolefin phosphates include those wherein the olefin group contains 2 or more carbon atoms. Polypeptides include polyaspartic and polyglutamic acids.

Azacycloalkane-2,2-diphosphonic acids are disclosed in US 3,941,772, issued March 2, 1976, Ploger et al., assigned to Henkel and US 3,988,443, issued Oct. 26, 1976, Ploger et al.

Optional agents to be used in place of or in combination with the pyrophosphate salt include such known materials as synthetic anionic polymers, including polyacrylates and copolymers of maleic anhydride or acid and methyl vinyl ether (e.g., Gantrez), as described, for example, in U.S. Patent 4,627,977, to Gaffar et al. ;as well as, e.g., polyamino propane sulfonic acid (AMPS), zinc citrate trihydrate, polyphosphates (e.g., tripolyphosphate; hexametaphosphate), diphosphonates (e.g., EHDP; AHP), polypeptides (such as polyaspartic and polyglutamic acids), and mixtures thereof.

### Antimicrobial Agents

Antimicrobial antiplaque agents may also by optionally present in the compositions. Such agents may include, but are not limited to, triclosan, 5-chloro-2-(2,4-dichlorophenoxy)-phenol, as described in The Merck Index, 11th ed. (1989), pp. 1529 (entry no. 9573) in U.S. Patent No. 3,506,720, and in European Patent Application No. 0,251,591 of Beecham Group, PLC, published January 7, 1988; chlorhexidine (Merck Index, no. 2090), alexidine (Merck Index, no. 222; hexetidine (Merck Index, no. 4624); sanguinarine (Merck Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; effective antimicrobial amounts of essential oils and combinations thereof for example citral, geranial, and combinations of menthol, eucalyptol, thymol and methyl salicylate; antimicrobial metals and salts thereof for example those providing zinc ions, stannous ions, copper ions, and/or mixtures thereof; bisbiguanides, or phenolics; antibiotics such as augmentin, amoxicillin, tetracycline, doxycycline, minocycline, and metronidazole; and analogs and salts of the above antimicrobial antiplaque agents; anti-fungals such as those for the treatment of *candida albicans.* If present, these agents generally are present in a safe and effective amount for example from 0.1% to 5% by weight of the compositions.

### Antiinflammatory Agents

Anti-inflammatory agents may also be present in the bleaching compositions. Such agents may include, but are not limited to, non-steroidal anti-inflammatory agents such as aspirin, ketorolac, flurbiprofen, ibuprofen, naproxen, indomethacin, aspirin, ketoprofen, piroxicam and meclofenamic acid, COX-2 inhibitors such as valdecoxib, celecoxib and rofecoxib, and mixtures thereof. If present, the anti-inflammatory agents generally comprise from 0.001% to 5% by weight of the bleaching compositions. Ketorolac is described in U.S. Patent 5,626,838, issued May 6, 1997.

### H-2 Antagonists

The bleaching compositions may also include a safe and effective amount of a selective H-2 antagonist. Selective H-2 antagonists include compounds which are disclosed in U.S. Patents 5,294,433 and 5,364,616 Singer et al., issued 3/15/94 and 11/15/94 respectively and assigned to Procter & Gamble, wherein the selective H-2 antagonist is selected from the group consisting of cimetidine, etintidine, ranitidine, ICIA-5165, tiotidine, ORF-17578, lupitidine, donetidine, famotidine, roxatidine, pifatidine, lamtidine, BL-6548, BMY-25271, zaltidine, nizatidine, mifentidine, BMY-25368 (SKF-94482), BL-6341A, ICI-162846, ramixotidine, Wy-45727, SR-58042, BMY-25405, loxtidine, DA-4634, bisfentidine, sufotidine, ebrotidine, HE-30-256, D-16637, FRG-8813, FRG-8701, impromidine, L-643728, and HB-408. Particularly preferred is cimetidine (SKF-92334), N-cyano-N'-methyl-N"-(2-(((5-methyl-1H-imidazol-4-yl)methyl)thio)ethyl)guanidine:

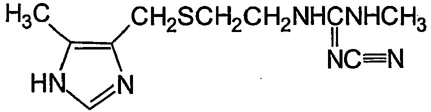

Cimetidine is also disclosed in the Merck Index, 11th edition (1989), p. 354 (entry no. 2279), and Physicians' Desk Reference, 46th edition (1992), p. 2228. Related preferred H-2 antagonists include burimamide and metiamide.

### Nutrients

Nutrients may improve the condition of the oral cavity and can be included in the bleaching compositions. Nutrients include minerals, vitamins, oral nutritional supplements, enteral nutritional supplements, and mixtures thereof.

Minerals that can be included with the compositions include calcium, phosphorus, fluoride, zinc, manganese, potassium and mixtures thereof. These minerals are disclosed in Drug Facts and Comparisons (loose leaf drug information service), Wolters Kluer Company, St. Louis, Mo., ©1997, pp10-17.

Vitamins can be included with minerals or used separately. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Such vitamins are disclosed in Drug Facts and Comparisons (loose leaf drug information service), Wolters Kluer Company, St. Louis, Mo., ©1997, pp. 3-10.

Oral nutritional supplements include amino acids, lipotropics, fish oil, and mixtures thereof, as disclosed in Drug Facts and Comparisons (loose leaf drug information service), Wolters Kluer Company, St. Louis, Mo., ©1997, pp. 54-54e. Amino acids include, but, are not limited to L-Tryptophan, L-Lysine, Methionine, Threonine, Levocamitine or L- carnitine and mixtures thereof. Lipotropics include, but, are not limited to choline, inositol, betaine, linoleic acid, linolenic acid, and mixtures thereof. Fish oil contains large amounts of Omega-3 (N-3) Polyunsaturated fatty acids, eicosapentaenoic acid and docosahexaenoic acid.

Antioxidants that may be included in the bleaching composition include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

Enteral nutritional supplements include, but, are not limited to protein products, glucose polymers, corn oil, safflower oil, medium chain triglycerides as disclosed in Drug Facts and Comparisons (loose leaf drug information service), Wolters Kluer Company, St. Louis, Mo., ©1997, pp. 55-57.

### Desensitizing Agents

Anti-pain or desensitizing agents can also be present in the bleaching compositions. Such agents may include, but are not limited to, strontium chloride, potassium nitrate, natural herbs such as gall nut, Asarum, Cubebin, Galanga, scutellaria, Liangmianzhen, Baizhi, etc.

### Optional Colorants

Dyes, pigments, colorants, and mixtures thereof may optionally be included in the compositions to give the compositions herein colored appearance. An advantage of adding pigments and/or colorants to the compositions herein is that it will allow the user to see if the composition covers their teeth evenly and completely, since coverage is easier to see with a colored composition. In one embodiment the colorant provides color similar to the color of natural teeth. Colorants useful herein are stable with the bleach agent and are those recognized as safe.

The levels of dye, pigments and colorants that are optionally used herein are in the range of 0.05% to 20%, in another embodiment from 0.10% to 15% and in another embodiment from 0.25% to 5% by weight of the composition.

### Methods of Manufacturing the Compositions

The integral carrier, such as a strip, may be formed by several of the film making processes known in the art. In one embodiment a strip of polyethylene is made by a blown process or a cast process. Other processes including extrusion or processes that do not affect the flexural rigidity of the strip of material are also feasible. Additionally, the second layer composition may be incorporated onto the strip during the processing of the strip. The second layer composition may be a laminate on the strip.

### Methods of Using the Compositions

The present invention can be applied to the teeth of a consumer in the dental office by a dental professional, or can be used at home by the consumer.

In practicing the present invention, the user applies the delivery system to one or more teeth. The composition is combined with an integral carrier such as a strip of material, dental tray, and/or sponge material, and thereafter applied to the teeth. In one embodiment, the delivery systems herein are almost unnoticeable when applied to the teeth.

Then, any residual composition may be easily removed by wiping, brushing or rinsing the oral surface after a desired period of time has elapsed, or in the normal course of tooth brushing or other oral care activities.

It is not necessary to prepare the teeth before applying the delivery systems of the present invention. For example, the user may or may not choose to brush the teeth or rinse the mouth before applying the present invention. The surfaces of the oral cavity are neither required to be dried nor to be excessively wet with saliva or water before application. However, it is believed that adhesion to the tooth enamel surfaces will be improved if the teeth are dry prior to application.

Where the integral carrier is a strip of material, the second layer composition may be coated on the strip of material, or be applied by the user to the strip of material, or be applied by the user to the teeth and then the strip of material placed over the coated teeth. The amount of the second layer composition applied to the strip of material or teeth may depend upon the size and capacity of the strip of material, concentration of the bleach agent and the desired benefit. Generally less than 1 gram of composition is required, in one embodiment from 0.001 grams to 0.0001 grams and in another embodiment from 0.1 gram to 0.4 grams of composition is used. The amount of composition per square centimeter (cm) of material is less than 1 gram/cm², in another embodiment less than 0.2 grams/cm², in another embodiment 0.0001 grams/cm² to 0.1 grams/cm², and in yet another embodiment 0.01 grams/cm² to 0.04 grams/cm².

The bleaching composition may be in the form of a viscous liquid, paste, gel, solution or other suitable form. In one embodiment the composition has a viscosity of from 200 to 1,000,000 cps at low shear rates (less than one 1/seconds). In another embodiment the viscosity is from 100,000 to 800,000 cps and in another embodiment from 400,000 to 600,000 cps.

The bleaching composition is a non-dentifrice, non-foaming, whitening composition, e.g. a whitening gel, wherein the whitening composition can for example, either be essentially free of foaming surfactants and, or essentially free of a fluoride ion source.

The present invention may allow for a decreased frequency of application. For example, a 6% peroxide containing composition that generally is used for 30 minutes twice daily for 2 weeks (e.g. for a total application time of 14 hours), may show substantially identical whitening efficacy by administering the same level of peroxide but used in accordance with the present invention, wherein the total application time is reduced to 6-10 hours. For example, when used in accord with the present invention the same level of bleach agent may achieve equal or similar efficacy with one 30 minute application per day for 14 days or a 30 minute application twice daily for 7-10 days.

Dental tray appliances may be used as follows. The patient or dental professional dispenses the present composition into a soft or rigid dental appliance and then the subject places the appliance over the subject's dental arch (or fits the device around his or her teeth to keep the tray in position). Generally, the recommended treatment period is the same as those discussed above. At the end of the treatment period, the dental appliance is removed, cleaned with water to remove any remaining composition, and then stored until the next application.

The compositions of this invention are useful for both human and other animals (e.g. pets, zoo, or domestic animals) applications.

### EXAMPLES

The following non-limiting examples further describe preferred embodiments within the scope of the present invention. Many variations of these examples are possible without departing from the scope of the invention.

### EXAMPLE I

The following compositions containing a bleaching agent, are made by processing techniques described below:

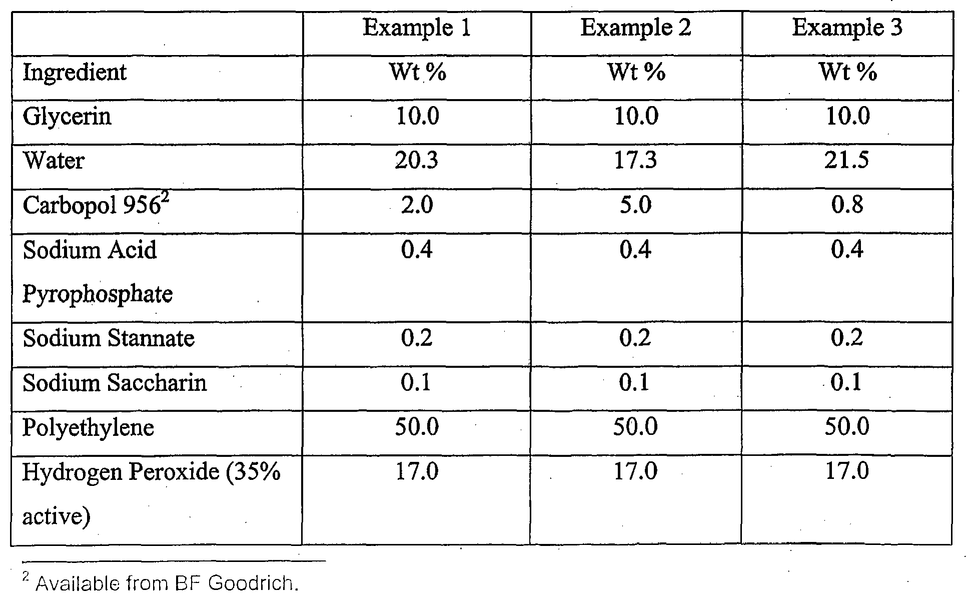

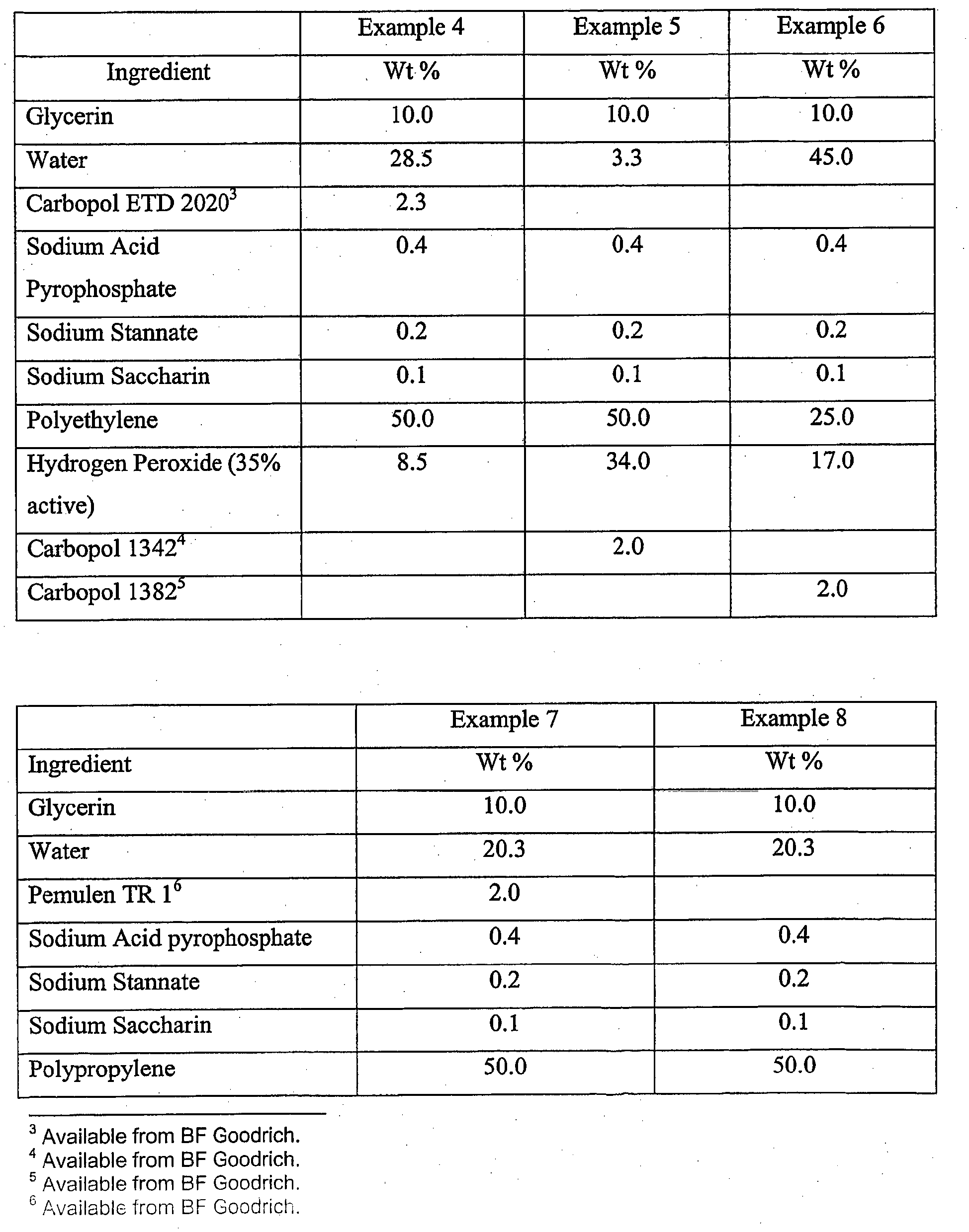

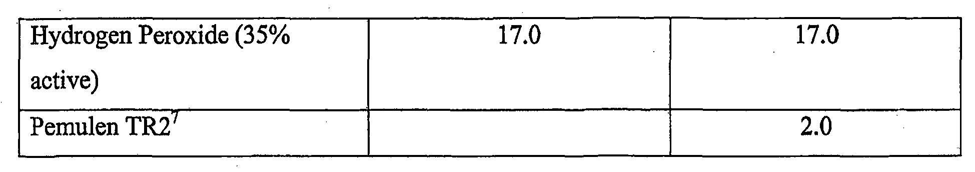

Examples 1-8 are made via the following process. Carbopol or Pemulen, glycerin, water, pyrophosphate, saccharin, stannate and hydrogen peroxide are mixed together at room temperature for 15-20 minutes at 150 rpms. The pH of the resulting mixture is then adjusted to about 4.5-5.0 by adding a 50/50 sodium hydroxide solution. Half of the desired amount of polyethylene is then mixed with the above mixture for about 10 minutes at room temperature at 150 rpms. The remaining half of the polyethylene is then added and mixed for another 10 minutes at room temperature at 150 rpms to provide the final gel formulation.

About 0.1-0.2g of the above composition may be applied directly to the teeth by any application methods disclosed herein. Alternatively the above compositions may be combined with an integral carrier such as a strip of material or tray and then applied to the teeth. About 0.1-0.2g or about 1-3 g of the above composition may be combined with a strip of material or tray, respectively. One example of a strip of material is a 0.013 mm thick piece of polyethylene film. The strip of material may be provided with an array of shallow pockets, typically 0.4 mm across and 0.1 mm deep. The strip of material has a flexural stiffness of about 0.6 grams/centimeter as measured on a Handle-O-Meter, model #211-300, available from Thwing-Albert Instrument Co. of Philadelphia, PA, as per test method ASTM D2923-95.

Any of the compositions described above can be used with any of the integral carriers described herein, e.g. strip of material, trays, and/or foam materials. In any of the above examples the polyethylene can be white or the color of natural teeth, allowing the user to apply a more even and continuous coating on the teeth. The above examples have a Whitening Index of from about 0.5 to about 4.

## Claims

1. An oral care delivery system (10) comprising:
a) an integral carrier (12) selected from a strip of material, a dental tray, sponge material and mixtures thereof; and
b) a bleaching composition (14) comprising:
i) from 0.1 % to 20% of a bleaching agent;
ii) from 30% to 75% of a water insoluble solid phase;
iii) from 20% to 75% of a water soluble liquid phase; and
iv) from 0.1 % to 10% of an associative thickener;
wherein the ratio of the water insoluble solid phase to the water soluble liquid phase is from 1:4 to 4:1 and wherein the water insoluble solid phase is selected from polyethylene, polypropylene, polyisoprenes, copolymers thereof, and mixtures thereof.

2. The delivery system of Claim 1 wherein the associative thickener is an acrylate/C10-C30 alkyl acrylate crosspolymer.

3. The delivery system of Claim 1 wherein the associative thickener is a hydrophobically modified nonionic polyol polymer.

4. The delivery system of Claim 1 wherein the water insoluble solid phase is polyethylene.

5. The delivery system of any of Claims 1 to 4 wherein the bleaching agent is selected from peroxides, metal chlorites, perborates, percarbonates, peroxyacids, persulfates, and combinations thereof.

6. The delivery system of any of Claims 1 to 5 wherein the composition comprises from 45% to 65% by weight of the water soluble liquid phase.

7. The delivery system of any of Claims 1 to 6 wherein the ratio of the water insoluble solid phase to the water soluble liquid phase is from 1: 1.5 to 1.5: 1, preferably from 1: 1.25 to 1.25: 1.

8. The delivery system of any of Claims 1 to 7 wherein the composition comprises from 45% to 60% by weight of the water insoluble solid phase.

## Patentansprüche

1. Mundpflegeabgabesystem (10), umfassend:
a) einen integralen Träger (12), der aus einem Materialstreifen, einer Zahnschiene, einem Schwammmaterial und Mischungen davon ausgewählt ist; und
b) eine Bleichmittelzusammensetzung (14), umfassend:
i) von 0,1 % bis 20 % ein Bleichmittel;
ii) von 30 % bis 75 % eine wasserunlösliche Festphase;
iii) von 20 % bis 75 % eine wasserlösliche Flüssigphase; und
iv) von 0,1 % bis 10 % ein assoziatives Verdickungsmittel;
wobei das Verhältnis der wasserunlöslichen Festphase zu der wasserlöslichen Flüssigphase von 1:4 bis 4:1 beträgt und wobei die wasserunlösliche Flüssigphase ausgewählt ist aus Polyethylen, Polypropylen, Polyisoprenen, Copolymeren und Mischungen davon.

2. Abgabesystem nach Anspruch 1, wobei das assoziative Verdickungsmittel ein Acrylat/C 1 0-C30-Alkylacrylatkreuzpolymer ist.

3. Abgabesystem nach Anspruch 1, wobei das assoziative Verdickungsmittel ein hydrophob modifiziertes nichtionisches Polyolpolymer ist.

4. Abgabesystem nach Anspruch 1, wobei die wasserunlösliche Festphase Polyethylen ist.

5. Abgabesystem nach einem der Ansprüche 1 bis 4, wobei das Bleichmittel ausgewählt ist aus Peroxiden, Metallchloriten, Perboraten, Percarbonaten, Peroxysäuren, Persulfaten und Kombinationen davon.

6. Abgabesystem nach einem der Ansprüche 1 bis 5, wobei die Zusammensetzung von 45 Gew.-% bis 65 Gew.-% die wasserlösliche Flüssigphase umfasst.

7. Abgabesystem nach einem der Ansprüche 1 bis 6, wobei das Verhältnis der wasserunlöslichen Festphase zu der wasserlöslichen Flüssigphase von 1:1,5 bis 1,5:1, vorzugsweise von 1:1,25 bis 1,25:1 beträgt.

8. Abgabesystem nach einem der Ansprüche 1 bis 7, wobei die Zusammensetzung von 45 Gew.-% bis 60 Gew.-% die wasserunlösliche Festphase umfasst.

## Revendications

1. Système d'administration de soins bucco-dentaires (10) comprenant :
a) un véhicule d'un seul tenant (12) choisi parmi une bande de matériau, une gouttière dentaire, un matériau de type éponge et leurs mélanges ; et
b) une composition de blanchiment (14) comprenant :
i) de 0,1 % à 20 % d'un agent de blanchiment ;
ii) de 30 % à 75 % d'une phase solide insoluble dans l'eau ;
iii) de 20 % à 75 % d'une phase liquide hydrosoluble ; et
iv) de 0,1 % à 10 % d'un épaississant associatif ;
dans lequel le rapport de la phase solide insoluble dans l'eau sur la phase liquide hydrosoluble va de 1:4 à 4:1 et dans lequel la phase solide insoluble dans l'eau est choisie parmi le polyéthylène, le polypropylène, des poly-isoprènes, leurs copolymères, et leurs mélanges.

2. Système d'administration selon la revendication 1, dans lequel l'épaississant associatif est un polymère réticulé acrylate/alkyl acrylate en C10 à C30.

3. Système d'administration selon la revendication 1, dans lequel l'épaississant associatif est un polymère de polyol non ionique rendu hydrophobe.

4. Système d'administration selon la revendication 1, dans lequel la phase solide insoluble dans l'eau est du polyéthylène.

5. Système d'administration selon l'une quelconque des revendications 1 à 4, dans lequel l'agent de blanchiment est choisi parmi des peroxydes, des chlorites métalliques, des perborates, des percarbonates, des peroxyacides, des persulfates, et leurs combinaisons.

6. Système d'administration selon l'une quelconque des revendications 1 à 5, dans lequel la composition comprend de 45 % à 65 % en poids de phase liquide hydrosoluble.

7. Système d'administration selon l'une quelconque des revendications 1 à 6, dans lequel le rapport de la phase solide insoluble dans l'eau sur la phase liquide hydrosoluble va de 1:1,5 à 1,5:1, de préférence de 1:1,25 à 1,25:1.

8. Système d'administration selon l'une quelconque des revendications 1 à 7, dans lequel la composition comprend de 45 % à 60 % en poids de la phase solide insoluble dans l'eau.
